# EUROPÄISCHE PATENTSCHRIFT

(11) **EP 1 803 462 B1**
(45) Veröffentlichungstag und Bekanntmachung des Hinweises auf die Patenterteilung: **01.11.2017**
(21) Anmeldenummer: 07101923.6
(22) Anmeldetag: 11.04.2003
(51) Int. Cl.: A61K 36/28, A61P 21/02

(54) **Verwendung von Petasites enthaltenden Zusammensetzungen zur Behandlung von Krankheitszuständen**
Application of compounds containing petasites for treating diseased states
Utilisation de compositions contenant du petasites pour le traitement d'états maladifs

(30) Priorität: 22.04.2002 DE 10217939
(43) Veröffentlichungstag der Anmeldung: 04.07.2007
(62) Teilanmeldung aus: 03727288.7
(73) Patentinhaber: Max-Zeller Söhne AG, 8590 Romanshorn (CH)
(72) Erfinder: Rittinghausen, Reiner, 82266, Inning/Ammersee (DE)
(74) Vertreter: Kasche, André

(56) Entgegenhaltungen:
- EP-A- 0 508 330
- EP-A- 0 547 465
- WO-A-00/12107
- WO-A-99/18984
- CH-A- 660 966
- DE-A- 4 002 938
- SIEGENTHALER P ET AL: "Sesquiterpenes from Petasites hybridus (Furanopetasin chemovar): Separation, isolation and quantitation of compounds from fresh plant extracts" PHARMACEUTICA ACTA HELVETIAE 1997 NETHERLANDS, Bd. 72, Nr. 2, 1997, Seiten 57-67, XP002253398 ISSN: 0031-6865
- DEBRUNNER B ET AL: "Sesquiterpenes of Petasites hybridus (L.) G.M. et Sch.: Influence of locations and seasons on sesquiterpene distribution" PHARMACEUTICA ACTA HELVETIAE 1995 NETHERLANDS, Bd. 70, Nr. 4, 1995, Seiten 315-323, XP002253399 ISSN: 0031-6865
- "PETASITES HYBRIDUS (BUTTERBUR)" ALTERNATIVE MEDICINE REVIEW, THORNE RESEARCH INC., SANDPOINT,, US, Bd. 6, Nr. 2, April 2001 (2001-04), Seiten 207-209, XP009016871 ISSN: 1089-5159
- DANESCH ULRICH ET AL: "Safety of a patented special butterbur root extract for migraine prevention." HEADACHE, Bd. 43, Nr. 1, 20. Januar 2003 (2003-01-20), Seiten 76-78, XP002253400 ISSN: 0017-8748
- CHIZZOLA REMIGIUS ET AL: "Variability in chemical constituents in Petasites hybridus from Austria" BIOCHEMICAL SYSTEMATICS AND ECOLOGY, Bd. 28, Nr. 5, Juni 2000 (2000-06), Seiten 421-432, XP002430772 ISSN: 0305-1978
- Hellmut Jork ET AL: "Kapitel 2.4 Papier- und Dünnschicht-Chromatographie" In: "Methodicum Chimicum, Band 1, Teile 1", 15 March 1973 (1973-03-15), Georg Thieme Verlag, Stutgart, XP055145655, ISBN: 978-3-13-480101-9 pages II-V, 67-79,
- "Kapitel 11.2, Sektion 2.2 Trennung durch Dünnschichtchromatographie" In: "Methodicum Chimicum, Band 1, Teile 1", 15 March 1973 (1973-03-15), Georg Thieme Verlag, Stutgart, XP055145660, ISBN: 978-3-13-480101-9 pages II-V, 915-920,
- Anonymous ET AL: "Chemiepraktikum für Biologen - SS 2002 - Versuchsprotokoll - Dünnschichtchromatographie", , 11 May 2011 (2011-05-11), pages 1-7, XP055146334, Mainz Retrieved from the Internet: URL:http://www.staff.uni-mainz.de/freesec/ Download/Chemie/DC_Amino_Monosacc.pdf [retrieved on 2014-10-14]
- Kai Jung: "Theoretische Grundlagen", , 16 November 2000 (2000-11-16), pages 1-3, XP055146348, Retrieved from the Internet: URL:http://www.reaktionen.org/node69.html [retrieved on 2014-10-14]

## Beschreibung

Die vorliegende Erfindung betrifft die Verwendung eines Petasitesextrakts und/oder von Petasitesextraktfraktionen, welche frei von Furanopetasinen sind,

Die Pflanze Petasites wird auch als Pestwurz bezeichnet. Petasitesextrakte bzw. Pestwurzextrakte können aus der Pflanze und/oder Pflanzenteilen gewonnen werden, wobei Blätter und/oder Wurzeln bevorzugt werden.

Petasitesextrakte werden üblicherweise besonders bevorzugt aus den Wurzeln der Pestwurz isoliert und haben eine krampflösende und analgetische Wirkung. Diese Wirkung der Pestwurz war bereits Hippokrates, Galen und Paracelsus bekannt.

In der DE-A1 198 38 848 wird offenbart, dass Petasitesextrakte zur Behandlung von gastrointestinalen Erkrankungen, von Asthma, von Pollinosis, von Dysmenorrhoe, von Ekzemen, von Migräne, von Psoriasis, von Bluthochdruck und/oder zur Verwendung als Spasmolytikum geeignet sind.

CH 660 966 beschreibt, dass Handelsextrakte aus Petasites hybridus mit einem Petasingehalt von 0,1 bis 5% bekannt sind. Die Veröffentlichung zielt auf die Erhöhung des Petasingehalts durch verbesserte Extraktionsemethoden ab: 60-75%, da die Petasine die spasmolytische Wirkung tragen. Es handelt sich um apolare Extrakte. Ferner wird auf die unterschiedlichen Isomere von Petasin hingewiesen, die durch Isomerisierung während der Extraktion entstehen können.

Bei den derzeit verwendeten pharmazeutischen Präparaten aus Petasitesextrakt handelt es sich insbesondere um zähflüssige Extrakte von Rhizoma Petasites, d.h. Extrakte aus dem Wurzelstock der Pestwurz, die einen unangenehmen, bitteren Geschmack aufweisen.

Die Pestwurz enthält neben Sesquiterpenen, Eremophilane auch Pyrrolizidinalkaloide. Den Pyrrolizidinalkaloiden ist das Pyrrolizidin-Grundgerüst gemeinsam. Sie sind weltweit in höheren Pflanzen verbreitet. So findet man Pyrrolizidinalkaloide und ihre N-Oxide unter anderem in den Gattungen der Petasites. Die in den Pflanzenteilen der Gattung Petasites enthaltenen Pyrrolizidinalkaloide zeichnen sich durch erhebliche hepatotoxische, carcinogene und mutagene, aber auch zytostatische Eigenschaften aus. Die Toxizität der Pyrrolizidinalkaloide ist an bestimmte Strukturgruppen gebunden, die folgende Strukturmerkmale aufweisen:
Doppelbindungen in der 1,2-Stellung des Pyrrolizidin-Ringes;
Veresterungen mindestens der primären Hydroxymethylgruppe mit einer C₅ oder C₆-Carbonsäure;
Verzweigung der Alkylseiten-Kette mit mindestens einer der Necinsäuren.

Die höchste Toxizität und Cancerogenität besitzen die cyclischen Dieser. Die Pyrrolizidinalkaloide werden nach peroraler Aufnahme rasch resorbiert, ihre N-Oxide erst nach Reduktion durch die Darmflora. Bei der Metabolisierung in der Leber werden die Pyrrolizidinalkaloide durch mischfunktionelle Oxidasen in sehr toxische Pyrrol-Derivate umgewandelt. Diese sind sehr reaktiv und alkylieren unter physiologischen Bedingungen nukleophile Gruppen der DNA, wie Amino-, Thiol- und Hydroxy-Gruppen.

In der Phytotherapie wurden pyrrolizidinalkaloidhaltige Arzneimittelpflanzen seit langem als Naturheilmittel verwendet. Da das Bundesgesundheitsamt (BGA) alle Arzneipflanzen und ihre Präparate, die toxische Pyrrolizidinalkaloide enthalten, als gesundheitlich bedenklich und potentiell krebsauslösend eingestuft hat, wurde in Folge dessen intensiv daran gearbeitet, pharmakologisch aktive Petasitesextrakte zur Verfügung zu stellen, die einen deutlich verringerten Gehalt an Pyrrolizidinalkaloiden aufweisen.

Verfahren zur Herstellung von Petasitesextrakten sind im Stand der Technik gut bekannt.

Aufgabe der vorliegenden Erfindung ist es, neue Indikationen für die Verwendung von Zusammensetzungen enthaltend:
- petasinfreie Petasitesextraktfraktion(en);
anzugeben.

Der Gegenstand der vorliegenden Erfindung betrifft die Verwendung von polarem Petasitesextrakt und/oder polaren Petasitesextraktfraktionen, der und/oder die petasinfrei ist/sind oder einen sehr geringen Petasingehalt und einen Rf-Wert (DC, Kieselgel 60, Laufmittel Toluol/Ethylacetat im Verhältnis 93:7) von 0 - 0,21 aufweist/ aufweisen, zur Herstellung eines Arzneimittels.

Bevorzugt ist ein Petasites- extrakt und/oder Petasitesextraktfraktion(en) mit einem geringen Petasitesgehalt, Besonders bevorzugt sind petasinfreie Petasitesextrakt und/oder Petasitesextraktfraktion(en).

Erfindungsgemäss bevorzugt ist eine Petasitesextraktfraktion(en) mit einem Petasin-Gewichtsgehalt von ≤ 5 Gew.-%, von ≤ 4 Cew.-%, von ≤ 2 Gew.-%, von ≤ 1 Gew.-%, von ≤ 0,5 Gew.%, von ≤ 0,1 Ges.-% oder von ≤ 0,05 Gew.-%, bezogen auf das Gesamtgewicht der jeweiligen Petasitesextraktfraktion(en).

Noch mehr bevorzugt ist ein Petasitesextrakt und/oder Petasitesextraktfraktion(en) mit einem Petasin-Gewichtsgehalt von ≤ 0,01 Ges.-%, von ≤ 0,005 Ges.-%, von ≤ 0,001 Ges.-%, von ≤ 0,0005 Gew.-% oder von ≤0,0001 Gew.-%, bezogen auf das Gesamtgewicht des jeweiligen Petasitesextrakts oder bezogen auf das Gesamtgewicht der jeweiligen Petasitesextraktfraktion(en).

Am meisten bevorzugt ist ein Petasitesextrakt und/oder Petasitesextraktfraktion(en) der kein Petasin oder weniger als 5 ppm, bezogen auf den jeweiligen Petasitesextrakt und/oder Petasitesextraktfraktion( en), aufweist.

Apolare Petasitesextraktfraktion(en) weisen einen hohen Anteil an lipophilen Stoffen, wie beispielsweise Petasine, auf. Als apolare Petasitesextraktfraktion(en) werden hierin beispielsweise Fraktion(en) bezeichnet, die einen Rf-Wert im Bereich von 0,32 bis I aufweisen.

Als polare Petasitesextraktfraktion(en) werden in der Erfindung Fraktion(en) bezeichnet, die petasinfrei oder einen sehr geringen Petasingehalt aufweisen. Als polare Petasitesextraktfraktion(en) werden in der Erfindung Fraktion(en) bezeichnet, die einen Rf-Wert im Bereich von 0 bis 0,21 aufweisen.

Polare Petasitesextraktfraktion(en) mit einem Rf-Wert im Bereich von 0,01 bis 0,20, vorzugsweise 0,02 bis 0,19, weiter bevorzugt 0,04 bis 0,17, noch weiter bevorzugt von 0,06 bis 0,15, insbesondere bevorzugt von 0,08 bis 0,13 sind erfindungsgemäss verwendbar.

Es können aber auch polare Petasitesextraktfraktion(en) mit einem Rf-Wert im Bereich von 0,03 bis 0,18, vorzugsweise 0,05 bis 0,16, weiter bevorzugt 0,07 bis 0,14, noch weiter bevorzugt von 0,09 bis 0,12, insbesondere bevorzugt von 0,10 bis 0,11 erfindungsgemäss verwendet werden.

Petasinhaltige Petasitesextraktfraktion(en) weisen im wesentlichen einen Rf-Wert im Bereich von ≥ 0,21 bis ≤ 0,32 auf.

Die R_{f}-Werte wurden ermittelt, indem manjeweils 5 µl Probe eines Petasitesextrakt und/oder Petasitesextraktfraktion(en), die als Lösemittel 1 Gew.-% Ethanol enthalten, bezogen auf das jeweilige Gesamtgewicht des Petasitesextrakt oder Petasitesextraktfraktion(en), auf eine Dünnschichtchromatographie DC-Alufolie Kieselgel 60 F254, erhältlich bei der Firma Merck, an der Startlinie, auch als Ausgangspunkt bezeichnet, aufbringt und ein Laufmittel Toluol/Ethylacetat im Verhältnis 93:7 verwendet. Die Dünnschichtchromatographie wurde nach Ausbildung einer Fliessmittelfront von 5,6 cm, gemessen von der Startlinie, abgebrochen. Die Durchführung der Dünnschichtchromatographie ist dem Fachmann gut bekannt und wird beispielsweise in Hans Rudolf Christen und Fritz Vögtle, Organische Chemie-von den Grundlagen zur Forschung-Band 1,36 - 37, 1988 beschrieben.

Die Detektion der chromatographisch getrennten Stoffe wurde mittels des dem Fachmann bekannten Detektionsmittel "Anisaldehyd /H₂SO₄" durchgeführt.

Erfindungsgemäss ist ein Petasitesextrakt und/oder Petasitesextraktfraktion(en) bevorzugt, wobei der Petasitesextrakt und/oder Petasitesextraktfraktion(en) weniger 5 Ges.-%, noch weiter bevorzugt weniger als 1 Ges.-%, besonders bevorzugt weniger als 0,1 Ges.-%, insbesondere weniger als 0,01 Gew,-%, und besonders bevorzugt weniger als 0,001 Gew.%, bezogen auf das Gesamtgewicht des lösemittelfreien Petasitesextrakt oder Petasitesextraktfraktion(en), aufweist.

Üblicherweise unterscheidet man die Petasitesextraktfraktion(en) in polare und nicht polare Petasitesextraktfraktion(en). Nicht polare Petasitesextraktfraktion(en) weisen einen hohen Anteil an lipophilen Stoffen, wie beispielsweise Petasine, auf. Hingegen sind polare Petasitesextraktfraktion(en) vorzugsweise petasinfrei oder weisen allenfalls einen geringen Anteil an Petasinen auf.

Ein polarer Petasitesextrakt und/oder Petasitesextraktfraktion(en) zeichnen sich durch einen verminderten Gehalt an Petasin bzw. Petasinen aus.

Die pharmakologisch wirksame Petasitesextrakt und/oder wenigstens eine Petasitesextraktfraktion enthaltende Zusammensetzung kann erfindungsgemäss insbesondere zur Behandlung von und/oder zur Prophylaxe von durch spezifische COX-2-Hemmung behandelbare Erkrankungen, umfassend:
Erkrankungen der Gelenke und des Bindegewebes, beispielsweise Arthritis, Arthrose, Osteoarthritis, Rheumatoider Arthritis, chronischer Polyarthritis, Bandscheibenleiden; und/oder
koronaren und/oder thromboembolischen Erkrankungen, beispielsweise entzündliche Herzkranzgefässe, Myokarditis, Myokardinfarkt, instabile und stabile Angina pectoris, transitorischer ischämischer Attacke, Apoplex, reversibles ischämisches neurologisches Defizit, prolongiertes ischämisches neurologisches Defizit, periphere arterielle Verschlusskrankheiten; und/oder
gastrointestinale Erkrankungen, beilspielsweise Colitis, Gastritis, Gastroduodenitis, Colitis ulcerosa, gastrointestinale Ulzerationen, exogene und endogene Schädigungen der Magen-Darmmukasa; und/oder
Polypen, Adenomen, beispielsweise im Darm, Adenomen in der Nase und/oder Adenomen in Nebenhöhlen; und/oder
Hauterkrankungen, beispielsweise Psoriasis, Ekzeme; und/oder
WS-Syndrom, Rückenschmerzen, Kopfschmerzen, Migräne, Schmerzen nach Zahnbehandlung, Schmerzen bei Krebskranken, Tumoranalgetikum, krampfartige Schmerzen im Magen-Darm-Trakt, krampfartige
Schmerzen im Urogenitalbereich, schmerzhafte Regelstörungen (Dysmenorrhoe); und/oder
Blasenerkrankungen, beispielsweise Reizblase, Zystitis, Tenesmen, Miktionsstörungen, Pollakisurie, Nykturie; und/oder
Behandlung von Tumoren, beispielsweise von Tumoren aufgrund von antiproliferativer Effekte; und/oder
von ≤ neuro-vegetativen Störungen, beispielsweise des Magens, insbesondere Reizmagen, der Gallenwege, insbesondere Dyskinesien der Gallenwege, Cholecystitis und /oder Cholangitis; und/oder
Inkontinenz, beispielsweise Stress-Inkontinenz, Drang-Inkontinenz, Reflex-Inkontinenz und/oder Überlauf-Inkontinenz,
Unruhezustände, beispielsweise Vegetative Störungen, Angstzustände, Schlafstörungen, Depressionen verabreicht werden.

Zusammensetzungen zur Verwendung in der vorliegenden Erfindung können ausgewählt sein aus der Gruppe umfassend:
Pharmazeutische und veterinärmedizinische Erzeugnisse; Sanitärprodukte für medizinische Zwecke; diätetische Erzeugnisse für medizinische Zwecke; Babykost; Pflaster; Verbandmaterial; Desinfektionsmittel; Nahrungsergänzungsmittel zu medizinischen und nichtmedizinischen Zwecken.

Besonders bevorzugte Zusammensetzungen zur Verwendung in der vorliegenden Erfindung sind ausgewählt aus der Gruppe Arzneimittel, Nahrungsergänzungsmittel zu medizinischen Zwecken.

Zusammensetzungen werden in dieser Erfindung auch als Mittel bezeichnet.

Derartige Zusammensetzungen können wenigstens 0,01 mg bis maximal 10 Ges.-% Petasitesextrakt und/oder Petasitesextraktfraktion( en), vorzugsweise maximal 5 Gew.-%. insbesondere maximal 1 Ges.-% und gegebenenfalls maximal 0,1 Gew.-%, bezogen auf das Gesamtgewicht der Zusammensetzung, aufweisen,

Der Petasitesextrakt oder Petasitesextraktfraktion( en) im Sinne dieser Erfindung bestehen aus einem komplexen Wirkstoff-Gemisch. Der Petasitesextrakt oder Petasitesextraktfraktion(en) kann als Wirkkomponente Sesquiterpene umfassen.

Gewichtsangaben beziehen sich, wenn nicht anders angegeben auf das Gesamtgewicht des Petasitesextrakts oder Petasitesextraktfraktion(en) frei von zugesetzten Lösemitteln oder Extraktionsmitteln. Frei von zugesetzten Lösemitteln oder Extraktionsmitteln bedeutet, dass ein möglicher verbleibender Restgehalt an zugesetzten Lösemittel im Petasitesextrakt oder Petasitesextraktfraktion 0 Ges.-% bis 1 Ges.-%, vorzugsweise weniger als 0,1 Ges.-%, bezogen auf das Gesamtgewicht des Petasitesextrakt oder Petasitesextraktfraktion, ausmacht.

Der Petasitesextrakt kann durch eine oder mehrere Petasitesextraktfraktionen angereichert sein. Hierdurch kann die Wirkung des Petasitesextrakt, durch Anreicherung mit bestimmten Petasitesextraktfraktion bzw, Petasitesextraktfraktionen verbessert werden.

Der Petasitesextrakt oder Petasitesextraktfraktion kann einen sehr geringen Gehalt an Petasinen umfassen.

Von Petasites gibt es 2 Chemovarietäten, die sich in der Art und Zusammensetzung der Petasine unterscheiden:
a) Furanopetasin-Chemovarietät mit Furanoeremophilanen, wie Furanoeremophilane, 9-hydroxy-furanoeremophilane, Furanopetasin, 2-Senecioyl-furanopetasol, 2-Tigloyl-furanopetasol, 2-Methlythioacryloyl-furanopetasol und/oder Eremophilanlactonen.
b) Petasin-Chemovarietät mit verschiedenen Petasinen, die aus Estern unterschiedlicher Säuren, wie veresterte Säuren: u.a. Angelicasäure, cis-3-methylthioacryloylsäure, Methacrylsaüre, 3-Methylcrotonsäure, Isobuttersäure mit den 3 isomeren Verbindungen Petasol, Isopetasol und/oder Neopetasol bestehen.

Die vorliegende Erfindung beschreibt die Verwendung einer Zusammensetzung, umfassend einen spezifischen COX-2-Hemmer basierend auf Petasitesextrakt und/oder Petasitesextraktfraktian(en).

Es wurde überraschend gefunden, dass ein petasinfreier oder petasinabgereicherter Petasitesextrakt und/oder Petasitesextraktfraktion(en) eine hohe spezifische COX-2-Hemmung verursacht, wobei ein petasinfreier oder petasinabgereicherter Petasitesextrakt und/oder Petasitesextraktfraktion(en) die höchste Aktivität hinsichtlich der COX-2-Hemmung zeigt.

Es wurde ferner überraschend gefunden, dass ein polarer Petasitesextrakt und/oder polarere Petasitesextraktfraktion(en) die Bildung von Prostaglandin E2 durch das Enzym Cyclooxygenase-2(COX-2) hemmt.

Es wurde ausserdem überraschend gefunden, dass der polarere Petasitesextrakt und/oder polarere Petasitesfraktion(en) ein spezifischer COX-2-Hemmer, der keine nachweisbare COX-1-Hemmung aufweist, ist.

Eine besonders starke spezifische COX-2-Hemmung wurde bei einem polareren Petasitesextrakt und/oder einer polareren Petasitesextraktfraktion(en) beobachtet.

Erfindungsgemäss zur Verwendung bevorzugt ist ein petasinfreier Petasitesextrakt oder eine petasinfreie Petasitesfraktion. Es können natürlich auch Extrakte oder Extraktfraktionen verwendet werden, die mit petasinfreien Petasitesfraktion(en) angereichert sind,

Dieser spezifische COX-2-Hemmer ist zur Behandlung und/oder Prophylaxe von durch spezifische COX-2-Hemmung behandelbare Krankheitszustände, vorzugsweise zur Behandlung und/oder Prophylaxe von wenigstens einer obengenannten Erkrankung verwendbar.

Der pharmazeutischen Zusammensetzung bzw, Mittel können weitere pharmazeutisch und/oder physiologisch wirksame Stoffe, wie Spurenelemente, zugesetzt werden

Weitere erfindungsgemäss vorteilhafte Ausführungsformen sind in den Unteransprüchen und in den Beispielen angegeben.

Geeignete Verfahren zur Herstellung von Petasitesextrakt sind in der DE 39 10 831, DE 41 11 141, DE 41 41 749 und WO 00/12107 beschrieben, auf die im vollem Umfang Bezug genommen wird.

Es gibt verschiedene Verfahren zur Herstellung von Petasitesextraktfraktionen.

Beispielsweise kann man einen Petasitesextrakt präparativ mittels bekannter Trennverfahren, wie Chromatographieverfahren, auftrennen;
a) über die Molekülgrösse
b) über die Polarität mittels Adsorptionschromatographie, wie DC oder HPLC

Chromatographische Trennverfahren lassen sich anhand der chemisch-physikalischen Eigenschaften der zu trennenden Stoffe und der technischen Umsetzung des Trennprozesses unterscheiden. Bis auf die Gelfiltration, die auf unterschiedlichen Transportwiderständen der Komponenten aufgrund der Molekülmassen und -größen beruht, nutzen chromatographische Verfahren die Adsorptionseigenschaften der Stoffkombination aus Trenngemisch, mobiler Phase und Adsorbens zur Trennung.

Das flüssige Stoffgemisch wird aufgrund der unterschiedlich intensiven Wechselwirkung seiner Komponenten mit einem Adsorbens, dem chromatographischem Material, in einer Trägerphase fraktioniert. Die mobile Phase durchströmt dabei das Adsorbens (inerte od. stationäre Phase). Zur Stofftrennung wird eine Probe des Gemisches in die mobile Phase eingebracht und durch das Adsorbens transportiert. Dabei wird die Stoffkomponente mit der größeren Affinität zum chromatographischen Material stärker zurückgehalten. Die mobile Phase wird so gewählt, dass sie zum einen die Durchströmung des Adsorbens und zum anderen das Herauslösen der adsorbierten Komponenten aus dem chromatographischen Material gewährleistet.

Je nach Beschaffenheit der zu trennenden Komponenten werden Gase, überkritische Fluide oder Flüssigkeiten als mobile Phasen eingesetzt. Mit der Flüssigchromatographie werden hochmolekulare Verbindungen sowie chemisch und physikalisch labile, polare, lipophile und nicht flüchtige Stoffe voneinander getrennt.

Im Stand der Technik wurde bisher der Petasitesextrakt aus dem Wurzelstock der Pestwurz (= Petasites) gewonnen. Nach der WO 00/12107 kann der Petasitesextrakt aus Pflanzen und/oder Pflanzenteilen, beispielsweise der Petasitesextrakt kann aus der Pflanze und/oder Pflanzenteilen der Gattung Petasites gewonnen werden.

Der Petasitesextrakt und/oder Petasitesextraktfraktion(en) können vorzugsweise aus Pflanzen und/oder Pflanzenteilen von Petasites hybridus, Petasites albus, Petasites japonicus, Petasites paradoxus, Petasites formosanus, Petasites kablikianus, Petasites tricholobus, Petasites niveus, Petasites amplus, Petasites georgicus, Petasites fragrans und/oder Petasites spurius gewonnen werden, wobei vorzugsweise die unterirdischen Pflanzenteile zur Herstellung des Extrakts verwendet werden.

Die Verwendung nicht nur der unterirdischen Pflanzenteile zur Herstellung des Petasitesextrakt und/oder Petasitesextraktfraktion(en), sondern auch anderer Pflanzenteile hat den Vorteil, dass man eine wesentlich höhere Ausbeute an Petasitesextrakt und/oder Petasitesextraktfraktion(en) bezogen auf die gesamte Pflanze erhält.

Der Petasitesextrakt oder Fraktion(en) davon sollten vorzugsweise frei von Pyrrolizidinalkaloiden und/oder Opinen sein.

Es wurde nunmehr gefunden, dass der Petasitesextrakt oder Fraktionen davon auch aus einer transformierten Pflanze, Pflanzenteilen, und/oder pflanzlichen Zellen, vorzugsweise mit Agrobacterium rhizogen transformierten Kulturen, insbesondere transformierten Wurzelhaarkulturen, gewonnen werden kann.

Bevorzugt ist die Gewinnung eines polareren Petasitesextrakt und/oder polarer Petasitesextraktfraktion(en).

Für diese biogenetische Herstellung lassen sich vorteilhaft sterile Sprosse oder andere Pflanzenteilen, z.B. juvenile Blütenteile von Petasites hybridus mit Agrobacterium rhizogenes transformieren. Hierdurch entstehen transformierte Wurzelhaarkulturen (hairy root cultures). Diese werden, beispielsweise in einem Fermentor, in einem Flüssig-Nährmedium kultiviert und vermehrt. Die Kulturen geben ihre Inhaltsstoffe unter anderem auch Petasine in das wässrige Medium ab, aus dem sie angereichert wurden.

Der nach einem biogenetischen Herstellungsverfahren erhaltene Petasitesextrakt kann gegebenenfalls Pyrrolizidinalkaloide und/oder Opinen aufweisen. Es ist daher vorteilhaft, die Kultur, die Petasine produzieren und am wenigsten Pyrrolizidinalkaloide und/oder Opinen aufweisen, zu selektieren.

Die Bildung bzw. Ausschleusung von Petasinen, bzw. generell Sesquiterpene oder Inhaltsstoffe kann mit sogenannten Elicitoren verstärkt werden.

Die Aufarbeitung, d.h. Isolierung und Anreicherung der Petasine aus dem Nährmedium, erfolgt nach üblichen dem Fachmann bekannten Verfahren.

Soweit bei der biogenetischen Herstellung Pyrrolizidinalkaloide und/oder Opinen gebildet werden, lassen sich diese nach mittels dem Fachmann gut bekannter Methoden abtrennen.

Bevorzugt ist, das der Petasitesextrakt und/oder Petasitesextraktfraktion(en) von zwischen ≥ 0 bis ≤ 5 ppm Pyrrolizidinalkaloid, vorzugsweise ≤ 1 ppm Pyrrolizidinalkaloid, besonders bevorzugt kein Pyrrolizidinalkaloid aufweist.

Es ist außerdem bevorzugt das der Petasitesextrakt und/oder Petasitesextraktfraktion(en) keine Opine aufweist. Opine sind seltene Aminosäuren, die von verschiedenen Agrobacterium-Stämmen nach Transfektion der entsprechenden Pflanzen gebildet werden. Die Gene für die Opine liegen auf einem Plasmid (Ti-Plasmid), einem DNA-Ring, der von dem Bakterium in die transfizierte Pflanze eingeschleust wird. In der vom Bakterium "befallenen" Pflanze wird dann das Opin gebildet und dient dem Bakterium als Nahrungsquelle.

Beispielsweise können bei der biogenetischen Herstellung im Petasitesextrakt und/oder Petasitesextraktfraktion(en) Opine enthalten sein. Es wird aber angestrebt, dass der erfindungsgemäss verwendbare Extrakt ≥ 0 bis ≤ 5 Gew.% Opine, vorzugsweise ≤ 1 Gew.-% Opine, bezogen auf das Gesamtgewicht des Extraktionsmittel freien Petasitesextrakt und/oder Petasitesextraktfraktion(en), aufweist.

Der Petasitesextrakt und/oder Fraktion davon kann in geringen Mengen Furanopetasine und/oder Petasine aufweisen.

Erfindungsgemäss verwendbare Petasitesextrakte oder Fraktionen davon können Furanopetasine und wenig oder keine Petasine enthalten.

Furanopetasin ist vorzugsweise ausgewählt aus der Gruppe, umfassend Furanoeremaphilane, wie 9-Hydroxy-furanoeremophilane, Furanopetasin, 2-Senecioyl-furanopetasol, 2-Tigloylfuranopetasol, 2-Methlythioacryloyl-furanopetasol und/oder Eremophilanlactone.

Petasin ist vorzugsweise ausgewählt aus der Gruppe, umfassend
- Petasinester, zusammengesetzt aus den Säurekomponenten, wie Angelicasäure, cis-3-methylthioacryloylsäure, Methacrylsäure, 3-Methylcrotonsäure,
- Isobuttersäure mit den Alkoholkomponenten, wie Petasol, Isopetasol und/oder Neopetasol,

Die pharmazeutische Wirkung von polaren Petasitesextrakt und/oder Petasitesextraktfraktion(en) konnte überraschenderweise auch durch Zusatz wenigstens eines pharmazeutisch und/oder physiologisch wirksamen Stoffs, umfassend wenigstens ein Antiphlogistikum bzw. Analgetikum, vorzugsweise Extrakte der Chamomilla recutita, Rhizoma Curcumae longae, Rhizoma Curcumae xanthorrhizae, Curcumae xanthorrhiza, Cortex Salicis, Salicis purpurea, Salicis daphenoides und/oder Tanacetum parthenium; wenigstens ein Spurenelement, vorzugsweise Salze von Eisen, Kobalt, Chrom, Iod, Kupfer, Mangan, Magnesium, Zink und/oder Selen; wenigstens ein Sekretolytikum bzw. Sekretomotorikum, vorzugsweise Extrakte der Süssholzwurzel, Thymiankraut und/oder Pfefferminzöl; wenigstens ein Bronchospasmolytikum, vorzugsweise Extrakte von ≤ Efeublättern; und/oder Vitamin, vorzugsweise Vitamin A, B, C, D und/oder E, gesteigert werden.

Ferner ist auch der Zusatz eines fiebersenkenden Mittels, wie Chrysanthemum Parthenium, auch als "Fever-few" bezeichnet, geeignet.

Die Resorption von pharmazeutischen Zusammensetzungen enthaltend einen polaren Petasitesextrakt und/oder Petasitesextraktfraktion(en) kann im Magen-Darmtrakt erhöht werden, wenn man dieser Zusammensetzung wenigstens einen Emulgator, wie Natriumalkylsulfat, Alkylsulfonat, Alkylcarboxylat, Alkylalkoholat, bevorzugt mit einer Alkylkettenlänge von C₁₂-C₁₈; Polyethylenglykolfettalkoholether, wenigstens einen Ester sowie Ether von Polyethylenglykol mit höheren Fettsäuren bzw, Fettalkoholen und/oder Polyethylenglykolstearat, besonders bevorzugt Cetylstearylalkohol und/oder Glycerin-polyethylenglycolricinoleat zusetzt.

Eine weitere erfindungsgemäss vorteilhafte Ausführungsform umfasst eine Zusammensetzung die einen polaren Petasitesextrakt und/oder Petasitesextraktfraktion(en) enthalten, die die Resorption im Magen-Darmtrakt verzögert.

Eine solche Zusammensetzung hat den Vorteil, dass man eine grössere Menge an Petasitesextrakt je Dosiseinheit verabreichen kann, die dann über einen verlängerten Zeitraum resorbiert wird. Hierdurch wird es möglich, einen praktisch konstanten Petasiteswirkstoff-Blutplasmaspiegel der pharmazeutisch aktiven Hauptwirkstoffe des Petasitesextrakts uber einen Zeitraum von wenigsten 12-48 Stunden, vorzugsweise 24 Stunden zu erreichen, der eine zweimalige, vorzugsweise einmalige Verabreichung der polaren Petasitesextrakt und/oder Petasitesextraktfraktion(en) enthaltenen Zusammensetzung am Tag erlaubt.

Geeignete Substanzen hierfür umfassen insbesondere die allgemein bekannten die Resorption im Magen-Darmtrakt für die Freisetzung von lipophilen Wirkstoffen verzögernden Stoffe. Vorzugsweise ist der die Resorption verzögernde Stoff, ausgewählt aus der Gruppe der Paraffine, vorzugsweise ein bei Raumtemperatur festes Paraffin, besonders bevorzugt ein Hartparaffin.

Ausserdem kann eine oral verabreichbare erfindungsgemässe einen polaren Petasitesextrakt und/oder Petasitesextraktfraktion(en) enthaltende Darreichungsform mit einer Magensaft resistenten und/oder die Resorption verzögernden Schicht überzogen werden.

Ein besonderer Nachteil des Petasitesextrakts/Fraktion ist dessen unangenehmer, bitterer Geschmack. In Folge dessen ist eine Darreichungsform, die den Wirkstoff bereits im Mund-/Rachenraum teilweise oder vollständig freisetzt, nicht möglich, da der Geschmack des Petasitesextrakts von den Patienten als extrem unangenehm empfunden wird und teilweise sofortige Übelkeit hervorruft.

Es wurde nun gefunden, dass der bittere, äusserst unangenehme Geschmack von Petasites bzw. Petasitesextrakt, durch Zusatz wenigstens eines geschmacksmaskierenden Stoffes wirksam maskiert werden kann, wobei der geschmacksmaskierende Stoff vorzugsweise ätherische Öle, Essenzen, Aromatische Wasser, Ölzucker, Fruchtaromen, wie Erdbeeraromen,

Zitrone, Vanille, und dergleichen, aromatische Drogenextrakte, künstliche Aromastoffe, Zucker, Polyole mit Süsskraft, neutral schmeckende Verdickungsmittel, Cyclodextrine und/oder Mischungen davon, umfasst.

Durch Zusatz eines geschmacksverbessernden Stoffes lassen sich so Petasitesextrakt enthaltende Tees, Kautabletten, Säfte und dergleichen verabreichen.

Die erfindungsgemässe einen polaren Petasitesextrakt und/oder Petasitesextraktfraktion(en) enthaltende pharmazeutische Zusammensetzung kann in einer flüssigen, festen oder liposomalen Darreichungsform vorliegen. Erfindungsgemäss vorteilhaft ist es, wenn die Darreichungsform ein Spray, ein Aerosol, ein Schaum, ein Inhalat, ein Pulver, eine Tablette, eine Kapsel, eine Weichgelatinekapsel, eine Kautablette, eine Salbe, eine Creme, ein Gel, ein Suppositorium oder eine Injektionslösung ist. Besonders bevorzugt ist es, wenn die erfindungsgemässe einen polaren Petasitesextrakt und/oder Petasitesextraktfraktion(en) enthaltende pharmazeutische Zusammensetzung als Tablette, Kapsel oder Kautablette, die den Wirkstoff in Form von Mikrokapseln umfasst, ausgebildet ist.

Als mögliche weitere pflanzliche Substanzen für eine erfindungsgemässe Petasitescreme sind insbesondere Calendula (tinctura/extraction), Viola (tinctura/extraction) und/oder Vitamine, vorzugsweise Vitamin E geeignet.

Erfindungsgemäß vorteilhaft ist es, wenn die jeweilige Einzeldosis der vorgenannten Darreichungsformen von 5 bis 100 mg Petasitesextrakt und/oder Petasitesextraktfraktion(en), vorzugsweise von 25 mg bis 100 mg Petasitesextrakt und/oder Petasitesextraktfraktion(en), bezogen auf den Lösemittel freien Petasitesextrakt und/oder Petasitesextraktfraktion(en), ausmacht.

Lösemittel im Sinne dieser Erfindung sind insbesondere Extraktionsmittel.

Besonders bevorzugt weist die Tagesdosis 5 - 600 mg Petasitesextrakt und/oder Petasitesextraktfraktion(en), weiter bevorzugt im Bereich von 10 - 500 mg Petasitesextrakt und/oder Petasitesextraktfraktion(en) und am meisten bevorzugt 250 mg Petasitesextrakt und/oder Petasitesextraktfraktion(en), bezogen auf den Lösemittel freien Petasitesextrakt und/oder Petasitesextraktfraktion(en), auf.

Außerdem ist die erfindungsgemäße Petasitesextrakt und/oder Petasitesextraktfraktion(en) enthaltende pharmazeutische Zusammensetzung zur Herstellung eines Arzneimittels zur Langzeitbehandlung bzw. Dauertherapie von Patienten, insbesondere Neugeborenen, Säuglingen und Kleinkindern geeignet. Es ist aber auch eine Akutbehandlung möglich, d.h. keine kurmäßige Anwendung, sondern unmittelbar bei oder nach Auftreten des Krankheitszustandes.

### Beispiel 1

| Zusammensetzung: | Qualität | Menge |
|---|---|---|
| Wirkstoff | | |
| polarere Petasitidisextraktfraktion frei von Petasin | | 25,0 mg |
| Hilfsstoffe | | |
| Mittelkettige Triglyceride* | Ph. Eur. 1997 | 245,0 mg |
| Kapselhülle | | |
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxid rot | E 172 | 0,47-0,55 mg |
| Glycerol | Ph. Eur. 1997 | 1,60-1,88 mg |
| Eisenoxid schwarz | E 172 | 1,13-1,33 mg |

| | | |
|---|---|---|
| * Weitere Lipoide, in denen sich der Extrakt löst, umfassen: Olivenöl, Maiskeimöl, Sojaöl. Therapeutische Plasmakonzentration 1 bis 100 mg/l. Ph. Eur. 1997 = Europäisches Arzneibuch 1997 E 172 = Eisenoxid (Farbstoffnummer für Arzneimittel) | | |

### Beispiel 2

**magensaftresistente Weichgelatinekapsel**

| Zusammensetzung: | Qualität | Menge |
|---|---|---|
| Wirkstoff | | |
| polarere Petasitidisextraktfraktion frei von Petasin | | 25,0 mg |
| Hilfsstoffe | | |
| Mittelkettige Triglyceride* | Ph. Eur. 1997 | 245,0 mg |
| Kapselhülle | | |
| Glycerol 85% | Ph. Eur. 1997 | 23,52-27,60 mg |
| Trockensubstanz aus Sorbitol-Lösung 70% Nicht kristallin | Ph. Eur. 1997 | 17,12-20,10 mg |
| Gelatine | Ph. Eur. 1997 | 80,89-94,96 mg |
| Eisenoxid rot | E 172 | 0,47-0,55 mg |
| Glycerol | Ph. Eur. 1997 | 1,60-1,88 mg |
| Eisenoxid schwarz | E 172 | 1,13-1,33 mg |
| Überzug | | |
| Celluloseacetatphthalat | | 1,0-15,0 mg |

| | | |
|---|---|---|
| * Weitere Lipoide, in denen sich der Extrakt löst, umfassen: Olivenöl, Maiskeimöl, Sojaöl. Ph. Eur. 1997 = Europäisches Arzneibuch 1997 E 172 = Eisenoxid (Farbstoffnummer für Arzneimittel) | | |

Therapeutische Plasmakonzentration 1 bis 100 mg/l.

### Beispiel 3

### Injektionslösung

Die polarere Petasitesextraktfraktion frei von Petasin wird 1:10 mit hochgereinigtem Sojaöl unter Zusatz von 0,1- 2 Gew.-% Eilecithin als Emulgator gemischt und die Mischung zu 0,5 bis 2,0 Gew.-% in Wasser für Injektionszwecke emulgiert.

Therapeutische Plasmakonzentration 1 bis 100 µg/l.

### Beispiel 4

### Liposomale Zusammensetzung

Die Liposomen werden aus Glycerophospholipiden, Cholesterol und Stearylamin und wenigstens einem Lipidderivat hergestellt, wobei die polarere Petasitidisextraktfraktion frei von Petasin(e) in der Lipidphase der Liposomen gelöst wird.

Therapeutische Plasmakonzentration 1 bis 100 mg/l.

### Beispiel 5

**Polare Petasitidisextraktfraktion frei von Petasin(e) in flüssiger Darreichungsform**

| Zusammensetzung 100g enthalten: | Qualität | Menge |
|---|---|---|
| Wirkstoff | | |
| polarere Petasitidisextraktfraktion | | 500 mg |
| Hilfsstoffe | | |
| Glycerin-polyethylenglycolricinoleat | USP XXII | 4500 mg |
| Ethanol 43% | HAB 1 | 95,000 g |
| | | 100,000 g |

Beispiele 6 bis 10 entsprechen den Beispielen 1 bis 5, mit dem Unterschied, das als Wirkstoff eine petasinfreie apolare Petasitidisextraktfraktion verwendet wurde.

### Verfahren zur Herstellung eines pyrrolizidinalkaloidfreien Petasitesextrakts

Zunächst wird mit Hilfe einer Durchlaufzentrifuge, wie in der DE 41 41 749 beschrieben, der Wassergehalt des Petasitesextrakt auf unter 1 Gew.-% abgesenkt. Anschließend wird der Extrakt mindestens einmal mit der ein- bis zehnfachen Menge an Säure ausgeschüttelt, die Säurephase abgetrennt, der Extrakt durch mehrmaliges Waschen auf einen pH-Wert von 3-7 gebracht und der Extrakt abschließend wieder auf einen Wassergehalt von 1 Gew.-% gebracht wird. Als Säure wird vorzugsweise eine Mineralsäure, besonders bevorzugt Schwefelsäure mit einer Normalität von 0,1 bis 1 verwendet. Der so erhaltene Extrakt hat einen Pyrrolizidinalkaloidgehalt von etwa 0,1 ppm. Dieser Extrakt wird dann in einem Nachbehandlungsschritt wenigstens 2 mal, vorzugsweise 4 bis 6 mal, im Verhältnis von 1 Teil Extrakt und 2 Teilen bidestilliertem Wasser, mit einer Wassertemperatur von wenigstens 40°C, vermischt. Nach jeder Durchmischung wird die Trennung der Wasserphase von der lipophilen Extraktphase durch Stehenlassen abgewartet und anschließend das überstehende Wasser von oben vorsichtig abgepumpt. Vorzugsweise wird im letzten Schritt emulgiertes Restwasser durch Zugabe von Natriumsulfat gebunden. Das mit Wasser beladene Natriumsulfat wird abzentrifugiert. Anschließend wurde ein ELISA zur Bestimmung des Restpyrrolizidinalkaloidgehalts im Petasitesextrakt durchgeführt, hierbei konnte kein Pyrrolizidinalkaloid mehr nachgewiesen werden. Die Nachweisgrenze eines ELISA für Pyrrolizidinalkaloide ist im Vergleich zur GC-MS-Kopplung wenigstens um den Faktor 1000 empfindlicher.

## Patentansprüche

1. Verwendung von polarem Petasitesextrakt und/oder polaren Petasitesextraktfraktionen, der und/oder die einen Rf-Wert (DC, Kieselgel 60, Laufmittel Toluol/Ethylacetat im Verhältnis 93:7) von 0 - 0,21 aufweisen, zur Herstellung eines Arzneimittels.

2. Verwendung nach Anspruch 1, **dadurch gekennzeichnet, dass** das Arzneimittel zur Behandlung und/oder Prophylaxe von durch spezifische COX-2-Hemmung behandelbare Erkrankungen verwendet wird, ausgewählt aus der Gruppe bestehend aus
(i) Erkrankungen der Gelenke und des Bindegewebes, vorzugsweise Arthritis, Arthrose, Osteoarthritis, rheumatoide Arthritis, chronische Polyarthritis, Bandscheibenleiden,
(ii) koronaren und/oder thromboembolischen Erkrankungen, vorzugsweise entzündliche Herzkranzgefäße, Myokarditis, Myokardinfarkt, instabile und stabile Angina pectoris, transitorische ischämische Attacke, Apoplex, reversibles ischämisches neurologisches Defizit, prolongiertes ischämisches neurologisches Defizit, periphere arterielle Verschlusskrankheit,
(iii) gastrointestinale Erkrankungen, vorzugsweise Colitis, Gastritis, Gastroduodenitis, Colitis ulcerosa, gastrointestinale Ulzerationen, exogene und endogene Schädigungen der Magen-Darmmukosa,
(iv) Polypen, Adenome, vorzugsweise im Darm, in der Nase und/oder in den Nebenhöhlen,
(v) Hauterkrankungen, vorzugsweise Psoriasis, Ekzeme,
(vi) WS-Syndrom, Rückenschmerzen, Kopfschmerzen, Migräne, Schmerzen nach Zahnbehandlung, Schmerzen bei Krebskranken, Tumorschmerzen, krampfartige Schmerzen im Magen-Darm-Trakt, krampfartige Schmerzen im Urogeni- talbereich, schmerzhafte Regelstörungen (Dysmenorrhoe),
(vii) Blasenerkrankungen, vorzugsweis Reizblase, Zystitis, Tenesmen, Miktionsstörungen, Pollakisurie, Nykturie,
(viii) Tumoren,
(ix) neuro-vegetative Störungen, vorzugsweise des Magens, mehr bevorzugt Reizmagen, der Gallenwege, mehr bevorzugt Dyskinesien der Gallenwege, Cholecystitis, Cholangitis,
(x) Inkontinenz, vorzugsweise Stress-Inkontinenz, Drang-Inkontinenz, Reflex-Inkontinez, Überlauf-Inkontinenz,
(xi) Unruhezustände, vorzugsweise vegetative Störungen, Angstzustände, Schlafstörungen, Depressionen,
(xii) Spasmen, spasmische Schmerzen.

3. Verwendung nach Anspruch 1 oder 2, **dadurch gekennzeichnet, dass** der Petasitesextrakt und/oder die Petasitesextraktfraktionen aus einer transformierten Pflanze, Pflanzenteilen und/oder pflanzlichen Zellen, vorzugsweise mit Agrobacterium rhizogen transformierten Kulturen davon, insbesondere transformierten Wurzelhaarkulturen, gewonnen werden.

## Claims

1. Use of a polar Petasites extract and/or polar Petasites extract fractions, having an Rf-value (thin layer chromatography, silica gel 60, solvent toluene/ethyl acetate in a ratio of 93:7) of 0-0.21, in the manufacture of a medicament.

2. Use according to claim 1, **characterized in that** the medicament is used in the treatment and/or prophylaxis of diseases that can be treated by specific COX-2-inhibition, selected from the group consisting of
(i) diseases of the joints and connective tissue, preferably arthritis, arthrosis, osteoarthritis, rheumatoid arthritis, chronic polyarthritis, intervertebral disc diseases,
(ii) coronary and/or thromboembolic diseases, preferably inflammatory coronary vessels, myocarditis, myocardial infarction, instable and stable angina pectoris, transient ischemic attack, apoplexy, reversible ischemic neurological deficit, prolonged ischemic neurological deficit, peripheral arterial occlusive disease,
(iii) gastrointestinal diseases, preferably colitis, gastritis, gastroduodenitis, colitis ulcerosa, gastrointestinal ulcerations, exogeneous and endogenous damages to the gastrointestinal mucosa,
(iv) polyps, adenomas, preferably in the intestine, in the nose and/or in the sinuses,
(v) skin diseases, preferably psoriasis, eczemas,
(vi) spinal syndrome, back pain, headache, migraine, pain after dental treatment, pain of cancer patients, tumor pain, spasmodic gastrointestinal pain, spasmodic pain in the urogenital region, painful menstruation disorder (dysmenorrhea),
(vii) bladder diseases, preferably irritable bladder, cystitis, tenesmus, micturition difficulties, pollakiuria, nocturia,
(viii) tumors,
(ix) neurovegetative disorders, preferably of the stomach, more preferably irritable stomach, of the bile tracts, more preferably dyskinesia of the bile tracts, cholecystitis, cholangitis,
(x) incontinence, preferably stress incontinence, urge incontinence, reflex incontinence, overflow incontinence,
(xi) restlessness, preferably vegetative disorders, anxiety, sleeping disorders, depressions,
(xii) spasms, spasmodic pain.

3. Use according to claim 1 or 2, **characterized in that** the Petasites extract and/or the Petasites extract fractions are obtained from a transformed plant, plant parts and/or plant cells, preferably cultures thereof that were transformed by Agrobacterium rhizogenes, in particular transformed root haircultures.

## Revendications

1. Utilisation d'extrait de pétasite polaire et/ou de fractions d'extrait de pétasite polaire qui présente et/ou présentent une valeur Rf (DC, gel de silice 60, éluant toluène/acétate d'éthyle dans le rapport 93:7) dans la plage de 0 à 0,21 pour la fabrication d'un médicament.

2. Utilisation selon la revendication 1, **caractérisée en ce que** le médicament est utilisé pour le traitement et/ou la prophylaxie de maladies traitées par inhibition spécifique de la COX-2, sélectionnée dans le groupe comprenant
(i) les maladies des articulations et du tissu conjonctif, principalement l'arthrite, l'arthrose, l'ostéroarthrite, la polyarthrite rhumatoïde, la polyarthrite chronique, la spondylose,
(ii) les maladies coronariennes et/ou thromboemboliques, principalement l'inflammation des artères coronaires, la myocardite, l'infarctus du myocarde, l'angine de poitrine stable et instable, l'accident ischémique transitoire, l'apoplexie, le déficit neurologique ischémique réversible, le déficit neurologique ischémique prolongé, l'artériopathie périphérique,
(iii) les maladies gastro-intestinales, principalement la colite, la gastrite, la gastroduodénite, la colite ulcéreuse, les ulcérations gastro-intestinales, les lésions exogènes et endogènes de la muqueuse gastrique et intestinale,
(iv) les polypes, les adénomes, principalement dans les intestins, dans le nez et/ou les sinus,
(v) les maladies de la peau, principalement le psoriasis, l'eczéma,
(vi) les pathologies de la colonne vertébrale, les douleurs dorsales, la céphalée, la migraine, les douleurs après un traitement dentaire, les douleurs chez les personnes atteintes d'un cancer, les douleurs tumorales, les douleurs spasmodiques dans l'appareil gastro-digestif, les douleurs spasmodiques dans la zone urogénitale, les menstruations douloureuses (dysménorrhée),
(vii) les maladies de la vessie, principalement la vessie irritable, la cystite, le ténesme, les troubles de la miction, la pollakiurie, la nycturie,
(viii) les tumeurs,
(ix) les troubles neuro-végétatifs, principalement de l'estomac, plus principalement la dyspepsie, des voies biliaires, plus principalement les dyskinésies des voies biliaires, la cholécystite, la cholangite,
(x) l'incontinence, principalement l'incontinence à l'effort, l'incontinence par impériosité, l'incontinence par réflexe, l'incontinence par regorgement,
(xi) les états d'agitation, principalement les troubles végétatifs, les états d'anxiété, les troubles du sommeil, les dépressions,
(xii) les spasmes, les douleurs spasmodiques.

3. Utilisation selon la revendication 1 ou 2, **caractérisée en ce que** l'extrait de pétasite polaire et/ou les fractions d'extrait de pétasite est et/ou sont issu et/ou issues d'une plante, de parties de plantes et/ou de cellules végétales modifiées, principalement de cultures modifiées avec Agrobacterium rhizogenes, notamment de cultures de poils absorbants modifiées.
